# EUROPEAN PATENT APPLICATION

(11) **EP 4 606 386 A2**
(43) Date of publication of application: **27.08.2025**
(21) Application number: 24218349.9
(22) Date of filing: 02.03.2018
(51) Int. Cl.: A61K 45/06

(54) **SPECIFIC INCREASE OF DOPAMINE SYNTHESIS THROUGH TARGETING OF THE GUANYLATE CYCLASE 2C RECEPTOR IN THE TREATMENT OF PARKINSON S DISEASE**

(30) Priority: 02.03.2017 EP 17158996
(62) Divisional of application: 18710566.3
(71) Applicant: Universiteit van Amsterdam, 1012 WX Amsterdam (NL)
(72) Inventor: VAN DER HEIDE, Lars Philip, 1012 WX Amsterdam (NL)
(74) Representative: V.O.

(57) **Abstract**

In embodiments the invention relates to means and methods for the treatment of Parkinson's disease. In some embodiments the means and methods involve a GUCY2C agonist. The invention also relates to test systems and cells that are suited to identify new candidate compounds for the treatment of Parkinson's disease.

## Description

The invention relates to means and methods for inducing and/or enhancing Guanylate Cyclase 2C signaling in cells and the use thereof in the production of dopamine by dopaminergic cells. The invention also relates to means and methods for the treatment of an individual that has Parkinson's disease or is at risk of developing this disease.

Parkinson's disease (PD) is the most prevalent neurological disorder after Alzheimer's pathology. Its primary hallmark is the progressive degeneration of dopaminergic neurons in the substantia nigra pars compacta (SNpc) (Kalia LV and Lang AE, 2015). This anatomical region arises from the mesencephalon during development and eventually creates projections towards the dorsal striatum, one of the forebrain areas by which the basal ganglia are comprised of (Smidt MP and Burbach JP, 2007; Baladron J and Hamker FH, 2015). The functional connectivity between both structures is broadly referred as the nigrostriatal pathway and depends on the release of dopamine (Ikemoto S et al., 2015). This neurotransmitter interacts with the striatal postsynaptic receptors after being released by the nigral efferents, ultimately regulating motor control. Accordingly, in PD patients in which there is a prominent loss of neurons in the SNpc, the resultant lack of dopamine release into the striatum triggers the onset of motor symptoms. Classically, these Parkinsonian manifestations can be classified into bradykinesia or slow body movement, tremor at rest, muscular stiffness and gait abnormalities (Kalia LV and Lang AE, 2015).

Nonetheless, motor impairments only appear when the degeneration in the SNpc occurs in an advanced stage, and they are preceded by a set of non-motor symptoms which often include olfactory dysfunction, depression or constipation (Mahlknecht P et al., 2015). The physiological cause of these early symptoms is not completely understood and, at least partially, is thought to be mediated by the malfunctioning of networks outside the nigrostriatal pathway and the imbalance of neurotransmitters apart from dopamine (Kalia LV and Lang AE, 2015). Interestingly, the latency between the outbreak of the prodromal phase and motor manifestations might be more than a decade (Postuma RB et al., 012). Therefore, the premotor period could offer physicians a temporal window which might be exploited to prevent the further development of the disease. Unfortunately, two major drawbacks arise when trying to employ this strategy to tackle PD: (1) non-motor symptoms are subtle to the clinical eye and not necessarily linked to this particular disease, and (2) the current comprehension of the molecular basis of PD is far from complete, rarely considers the underlying etiology of the disorder and often concedes a complex interplay of environmental and genetic factors (Kalia LV and Lang AE, 2015; Mahlknecht P et al., 2015).

Dopamine biosynthesis is complex and integrated with various other biosynthesis pathways. Dopamine is produced from L-tyrosine in two reactions. The enzyme tyrosine hydroxylase (Th) converts L-tyrosine into L-dopa and aromatic L-amino acid decarboxylase (AADC) converts L-dopa to form dopamine (Clarke CE, 2004). Both enzymes are also known to participate in the synthesis of two additional catecholamines (i.e. norepinephrine and epinephrine), whereas only the latter is shared with serotonin production (Purves D et al., 2001). Additionally, a negative feedback loop regulated by the amount of neurotransmitter negatively regulates the activity of the enzymes. Moreover, Th acts as the rate limiting step in the synthesis pathway. Dopamine interacts with the catalytic region of Th to decrease its activity (Dunkley PR et al., 2004). Dopamine is also subject to degradation. The enzymes catechol-O-methyltransferase (COMT) and monoamine oxidase B (MAOB) are involved in the degradation of dopamine and convert it to homovanillic acid (Clarke CE, 2004).

The most common drugs for Parkinson's disease are: levodopa; levodopa combined with peripheral AADC inhibitors (e.g. carbidopa); MAOB inhibitors, and COMT inhibitors (Kalia LV and Lang AE, 2015). These medicines can be taken orally and are able to cross the blood-brain barrier (BBB) with the exception of carbidopa, which cannot reach the central nervous system (CNS) (Ahlskog JE et al., 1989; Gershanik OS, 2015). A drawback of these treatments is that they exhibit (severe) side effects. These are thought to be caused among others by the fact that the drugs are not specific enough.

For instance, it is known that the drugs affect dopaminergic neurons outside the SNpc such as those in the ventral tegmental area or some hypothalamic nuclei (Upadhya MA et al., 2016). Stimulation of dopamine production in these of target cells can lead to side-effects of the treatment. Furthermore, the dopamine biosynthesis enzymes also participate in the production of compounds other than dopamine. Levodopa treatment can interfere with the synthesis of these other compounds and exert off-target effects in noradrenergic and serotonergic neurons (Carta M et al., 2007; Navailles S et al., 2014).

The unspecificity of the available drugs targeting PD triggers inescapable side effects, with levodopa-induced dyskinesias standing out the most. Orally-administered levodopa can cross the BBB and, once in the brain, be incorporated into the dopaminergic terminals of the dorsal striatum. There, it is further converted to dopamine by AADC, imported into vesicles by the transporter vMAT2 and ultimately released to the synaptic cleft. This outcome of levodopa treatment accounts for its beneficial effects in patients with PD. However, levodopa can also be incorporated into the striatal serotonergic terminals, in which AADC is equally present. It has been reported that dopamine, or 'false serotonin', can be synthesized and further released from serotonergic terminals in an activity-dependent manner upon levodopa administration. In elegant experiments performed in 6-OHDA-induced parkinsonian rats, levodopa-derived dyskinesias could be abolished by removing the serotonergic terminals in the dorsal striatum, or by silencing the neurotransmitter release with agonists of serotonin auto-receptors (Carta M et al., 2007).

It is an object of the present invention to provide means and methods for the treatment of Parkinson's disease that has less side-effects. It is a further object of the invention to use known compounds and identify new compounds for the treatment of Parkinson's disease. A treatment of the invention is particularly effective in the early stages of Parkinson's disease.

### Summary of the invention

The invention provides a GUCY2C agonist for use in the treatment of an individual that has Parkinson's disease.

The invention further provides a method of increasing dopamine production by a dopaminergic cell, the method comprising increasing signaling by Guanylate Cyclase 2C (GUCY2C) in said cell.

Also provided is a method of increasing the level of phosphorylation of Ser40 of tyrosine hydroxylase in a dopaminergic cell, the method comprising increasing signaling by GUCY2C in said cell.

The invention further provides an isolated or recombinant human cell comprising ectopic expression of human GUCY2C and/or ectopic expression of human tyrosine hydroxylase.

Also provided is a method for identifying a candidate compound for modifying dopamine production by a dopaminergic cell, the method comprising culturing an isolated or recombinant human cell comprising ectopic expression of human GUCY2C and/or ectopic expression of human tyrosine hydroxylase; contacting said cell with the candidate compound and determining the activity of tyrosine hydroxylase in said cell.

Further provided is a method for identifying a candidate compound for modifying dopamine production by a dopaminergic cell, the method comprising culturing an isolated or recombinant human cell comprising ectopic expression of human GUCY2C and/or ectopic expression of human tyrosine hydroxylase; contacting said cell with the candidate compound and determining whether signaling by GUCY2C has increased in said cell.

The invention also provides the use of a GUCY2C gene, an RNA or a protein encoded by the gene as a target for identifying a compound that is active in modulating tyrosine hydroxylase activity in a dopaminergic cell.

Also provided is a method of treatment of an individual that has Parkinson's disease, or is at risk of developing the disease, the method comprising administering a GUCY2C agonist to the individual in need thereof.

### Detailed description of the invention

Guanylate Cyclase 2C (GUCY2C ) is a member of the guanylyl cyclase family. Various aspects of the structure and function of the protein are described by Vaandrager (Mol. Cell. Biochem. 2002; 230:73-83). The expression of the gene and the function of the protein are highly regulated and dependent on the particular cell/tissue studied.

The GUCY2C gene and the protein encoded by the gene are known under a number of aliases, among which STA Receptor; Intestinal Guanylate Cyclase; Guanylyl Cyclase C; EC 4.6.1.2; GUC2C; HSTAR; STAR; GC-C; Guanylate Cyclase 2C; Heat Stable Enterotoxin Receptor; EC 4.6.1; DIAR6; MECIL; and MUCIL. External Ids for GUCY2C gene/protein are HGNC: 4688; Entrez Gene: 2984; Ensembl: ENSG00000070019; OMIM: 601330 and UniProtKB: P25092.

Tyrosine hydroxylase or tyrosine 3-monooxygenase is the enzyme responsible for catalyzing the conversion of the amino acid L-tyrosine to L-3,4-dihydroxyphenylalanine (L-DOPA). L-DOPA is a precursor for dopamine, which, in turn, is a precursor for the important neurotransmitters norepinephrine (noradrenaline) and epinephrine (adrenaline). Tyrosine hydroxylase catalyzes the rate limiting step in this synthesis of catecholamines. In humans, tyrosine hydroxylase is encoded by the TH gene, and the enzyme is present in the central nervous system (CNS), peripheral sympathetic neurons and the adrenal medulla. The protein or gene is known under a number of different names such as Tyrosine 3-Hydroxylase; EC 1.14.16.2; TYH; Dystonia; EC 1.14.16; DYT14; and DYT5b. External Ids for the TH Gene or protein are HGNC: 11782; Entrez Gene: 7054; Ensembl: ENSG00000180176; OMIM: 191290 and UniProtKB: P07101.

GUCY2C signaling is mediated through increased cellular cyclic guanosine-3',5'-monophosphate (cGMP). There are many downstream mediators of cGMP which include, depending on among others, the cell type, tissue and/or metabolic state thereof: intracellular phosphodiesterases (PDE), protein kinases (PK) and membrane-bound proteins and ion channels. When herein reference is made to GUCY2C signaling in the context of a dopaminergic neuron, reference is made to increased cGMP.

A dopaminergic cell is a cell that can produce dopamine. Such cells are typically specialized neuronal cells. Such neuronal cells are typically characterized by histochemical fluorescence detection of dopamine in the cells. Various groups of dopaminergic cells can be discriminated. Cell group A8 is one of such groups it is a small group of dopaminergic cells in rodents and primates. It is located in the midbrain reticular formation dorsolateral to the substantia nigra at the level of the red nucleus and caudally. In the mouse it is identified with the retrorubral field as defined by classical stains. Cell group A9 is a densely packed group of dopaminergic cells, and is located in the ventrolateral midbrain of rodents and primates. It is for the most part identical with the pars compacta of the substantia nigra as defined on the basis of Nissl stains. Cell group A10 is a large group of dopaminergic cells in the ventral midbrain tegmentum of rodents and primates. The cells are located for the most part in the ventral tegmental area, the linear nucleus and, in primates, the part of central gray of the midbrain located between the left and right oculomotor nuclear complexes. The dopaminergic cells of the claims are dopaminergic cells that express the GUCY2C receptor, such as preferably the group A8, group A9 or group A10 cells described above. Various other dopaminergic cells are known, some of which are listed herein below. Group A11 is a small group of dopaminergic cells located in the posterior periventricular nucleus and the intermediate periventricular nucleus of the hypothalamus in the macaque. In the rat, small numbers of cells assigned to this group are also found in the posterior nucleus of hypothalamus, the supramammillary area and the reuniens nucleus. Group A12 is a small group of cells in the arcuate nucleus of the hypothalamus in primates. In the rat a few cells belonging to this group are also seen in the anteroventral portion of the paraventricular nucleus of the hypothalamus. Group A13 is distributed in clusters that, in the primate, are ventral and medial to the mammillothalamic tract of the hypothalamus; a few extend into the reuniens nucleus of the thalamus. In the mouse, A13 is located ventral to the mammillothalamic tract of the thalamus in the zona incerta. Group A14 has a few cells observed in and near the preoptic periventricular nucleus of the primate. In the mouse, cells in the anterodorsal preoptic nucleus are assigned to this group. Group A16 is located in the olfactory bulb of vertebrates, including rodents and primates. Group Aaq is a sparse group of cells located in the rostral half of the central gray of the midbrain in primates. It is more prominent in the squirrel monkey (Saimiri) than the macaque. Various other groups exist in primates mice and other species (Felten et al (1983). Brain Research Bulletin. 10 (2): 171-284 and Dahlstrom A et al 1964). Acta Physiologica Scandinavica. 62: 1-55. Dopaminergic cells can also be generated artificially. Artificial cells can be provided with the necessary enzymes, receptor and/or coding regions therefore. In the context of the present invention HEK cells have been provided with Th and/or GUCY2C. The dopaminergic cell is preferably a dopaminergic neuronal cell. The dopaminergic cells is preferably a midbrain or a striatum cell. The dopaminergic cell is preferably a dopaminergic neuronal cell that expresses GUCY2C on the cell membrane. Preferably a substantia nigra cell. Neuronal cells can have very large projections, both incoming and outgoing projection. The cell is typically allocated to a section of the brain depending on the presence of the presence of the nucleus of the cell. For instance a neuronal cell of the midbrain has a cell part with the nucleus located in the midbrain. The cell can have projections such as axons or dendrites that pass through and/or end in other parts of the brain such as the striatum.

A phosphodiesterase (PDE) is an enzyme that breaks a phosphodiester bond. The term usually refers to cyclic nucleotide phosphodiesterases. However, there are many other families of phosphodiesterases, including phospholipases C and D, autotaxin, sphingomyelin phosphodiesterase, DNases, RNases, and restriction endonucleases (which all break the phosphodiester backbone of DNA or RNA), as well as numerous less-well-characterized small-molecule phosphodiesterases.

The cyclic nucleotide phosphodiesterases comprise a group of enzymes that degrade the phosphodiester bond in the second messenger molecules cAMP and cGMP. They regulate the localization, duration, and amplitude of cyclic nucleotide signaling within subcellular domains. PDEs are therefore important regulators of signal transduction mediated by these second messenger molecules.

The PDE nomenclature signifies the PDE family with an Arabic numeral, then a capital letter denotes the gene in that family, and a second and final Arabic numeral then indicates the splice variant derived from a single gene (e.g., PDE1C3: family 1, gene C, splicing variant 3). The superfamily of PDE enzymes in mammals is classified into 12 families, namely PDE1-PDE12. Different PDEs of the same family are functionally related despite the fact that their amino acid sequences can show considerable divergence. PDEs have different substrate specificities. Some are cAMP-selective hydrolases (PDE4, 7 and 8); others are cGMP-selective (PDE5, 6, and 9). Others can hydrolyse both cAMP and cGMP (PDE1, 2, 3, 10, and 11).

A phosphodiesterase inhibitor is a drug that blocks one or more of the subtypes of the enzyme phosphodiesterase (PDE), thereby preventing the inactivation of the intracellular second messengers cyclic adenosine monophosphate (cAMP) and cyclic guanosine monophosphate (cGMP) by the respective PDE subtype(s). Various selective and non-selective PDE inhibitors are known. Among the non-selective PDE inhibitors are caffeine; aminophylline; IBMX (3-isobutyl-1-methylxanthine); paraxanthine; pentoxifylline; and theophylline. These compounds are said to be non-selective, however, there activity may still vary. This can be the result of differences in preference; in affinity; and/or in pharmacokinetics, among others. In some embodiments the PDE inhibitor is a non-selective inhibitor such as IBMX.

The guanylin family of peptides has 3 subclasses of peptides containing either 3 intramolecular disulfide bonds found in bacterial heat-stable enterotoxins (ST), or 2 disulfides observed in guanylin and uroguanylin, or a single disulfide exemplified by lymphoguanylin. These peptides bind to and activate cell-surface receptors that have intrinsic guanylate cyclase (GC) activity such as GUCY2C.

Guanylin is a natural agonistic ligand of GUCY2C. It is a 15 amino acid polypeptide. It is secreted by goblet cells in the colon. Guanylin acts as an agonist of the guanylyl cyclase receptor GC-C and regulates electrolyte and water transport in intestinal and renal epithelia. Upon receptor binding, guanylin increases the intracellular concentration of cGMP, induces chloride secretion and decreases intestinal fluid absorption, ultimately causing diarrhea. The peptide stimulates the enzyme through the same receptor binding region as the heat-stable enterotoxins. It is known under a number of names some of which are guanylate cyclase activator 2A; Guanylate Cyclase-Activating Protein 1; Guanylate Cyclase-Activating Protein I; Prepro-Guanylin; GCAP-I; GUCA2; Guanylate Cyclase Activator 2A; and STARA. External Ids for the guanylin gene/protein are HGNC: 4682; Entrez Gene: 2980; Ensembl: ENSG00000197273; OMIM: 139392; and UniProtKB: Q02747.

Uroguanylin a natural agonistic ligand of GUCY2C. It is a 16 amino acid peptide that is secreted by cells in the duodenum and proximal small intestine. Guanylin acts as an agonist of the guanylyl cyclase receptor GC-C and among others regulates electrolyte and water transport in intestinal and renal epithelia. In humans, the uroguanylin peptide is encoded by the GUCA2B gene. The gene and protein are known under a number of different names Guanylate Cyclase Activator 2B; Prepro-Uroguanylin; GCAP-II; and UGN. External Ids for the gene and protein are HGNC: 4683; Entrez Gene: 2981; Ensembl: ENSG00000044012; OMIM: 601271; and UniProtKB: Q16661.

Lymphoguanylin is a natural agnostic ligand of GUCY2C. It is among others described in Forte et al. Endocrinology. 1999 Apr;140(4):1800-6.

A functional derivative of guanylin, lymphoguanylin or uroguanylin has the same activity in kind not necessarily in amount. A functional derivative preferably has the same amino acid sequence as guanylin, uroguanylin or STh or has an altered amino acid sequence which is highly similar to guanylin, uroguanylin or STh. One such derivative is linaclotide. Linaclotide is a peptide mimic of endogenous guanylin and uroguanylin. It is a synthetic tetradecapeptide (14 amino acid peptide) with the sequence CCEYCCNPACTGCY (H-Cys-Cys-Glu-Tyr-Cys-Cys-Asn-Pro-Ala-Cys-Thr-Gly-Cys-Tyr-OH). Linaclotide has three disulfide bonds which are between (when numbered from left to right) Cys1 and Cys6, between Cys2 and Cys10, and between Cys5 and Cys13;[9]. Three similar peptide agonists of GUCY2C are in clinical development: linaclotide (Linzess^{™}, Forest Laboratories and Ironwood Pharmaceuticals, Inc.); SP-304 (plecanatide) and SP-333 (Synergy Pharmaceuticals, Inc.). A functional derivative can have a chemical group attached to the N- and/or C-terminal end. The chemical group can have one or two amino acids in peptide linkage. The functional derivative may be derived from guanylin or uroguanylin by chemical modification of one or more of the amino acid residue side chains. One such modification or chemical group may be a modification to further facilitate passage of the blood brain barrier. Guanylin that is injected into the blood stream rapidly enters the brain (WO2013016662). Guanylin is capable of passing the blood-brain barrier (BBB). Brain delivery can be a challenge in drug development. Although the blood-brain barrier prevents many drugs from reaching their targets, molecular vectors - known as BBB shuttles - offer great promise to safely overcome this formidable obstacle. In recent years, peptide shuttles have received growing attention because of their lower cost, reduced immunogenicity, and higher chemical versatility than traditional Trojan horse antibodies and other proteins. Suitable BBB shuttles are described in Oller-Salvia et al (Chem. Soc. Rev., 2016, 45, 4690-4707) which is incorporated by reference herein for this purpose. In a preferred embodiment the functional guanylin or uroguanylin derivative is guanylin or uroguanylin fused to a peptide BBB shuttle of table 1 of Oller-Salvia et al. The fusion is typically done by means of a peptide linkage. A linker can be introduced between the two functional units. The linker is preferably a peptide linker of 1-20, preferably 1,15, preferably 1-10 and more preferably 1-5 amino acid residues.

A GUCY2C ligand is a molecule that binds to an extra-cellular part of GUCY2C and that modulates activity of the receptor. A ligand that increases signaling of the receptor is referred to as a GUCY2C agonist. Signaling is typically increased to the level of a natural ligand of the receptor. Activation is preferably at least 50% of the level of activation achieved by a natural ligand of the receptor tested under otherwise identical conditions (of course under sufficiently saturating conditions). The level of activity of signaling is typically measured by measuring the production of intracellular cGMP. It can also be measured by measuring the level of an activity resulting from the level of cGMP in the cell.

A GUCY2C agonist is a molecule that binds to an extra-cellular part of GUCY2C and increases signaling by the protein. Increasing in this context includes the induction of signaling by a silent GUCY2C receptor and an increase of signaling on top of an already existing signaling activity of the receptor. Induction or increase of an existing signaling activity is typically measured by comparing the integrated results of a number of cells, typically a pool of at least 10.000 cells. In such cases induction of signaling is a signal from undetectable to detectable. An increase on top of an existing activity is a higher activity compared to the absence of the agonist under otherwise identical conditions. An increase of signaling thus includes an increase from undetectable to detectable signaling levels.

Various agonists are known in the art. Most well-known are the members of the guanylin family of peptides. These include for instance the peptides mentioned above and the ST bacterial peptides such as the *E*. *coli* STa; the *V. cholera* ST and the *Y*. *enterocolitica* (Fonteles et al 2011; Can. J. of Phys. And Pharmac. 89: 575-85). US8,748,575 (Wolfe et al) describes guanylin and uroguanylin proteins from various species. Wolfe et al also describe various guanylin and uroguanylin derivatives that are GUCY2C agonists. Also described are various bacterial peptides that mimic the action of the peptides mentioned above. These peptides and mutants thereof are GUCY2C agonists. US2012/0108525 (Curie et al) describe various GUCY2C agonists and the use of these agonists in the treatment of gastrointestinal disorders. WO2013016662 (Ganat et al) describes guanylin-conjugates having a payload to target the payload to the brain. It in particular describes the use of the payload L-Dopa.

Dopamine biosynthesis is complex and integrated with various other biosynthesis pathways. Dopamine is produced from L-tyrosine in two reactions. The enzyme tyrosine hydroxylase (Th) converts L-tyrosine into levodopa (L-dopa) and aromatic L-amino acid decarboxylase (AADC) decarboxylates levodopa to form dopamine by (Clarke CE, 2004). Tyrosine Hydroxylase is known under a number of aliases Tyrosine 3-Monooxygenase; Tyrosine 3-Hydroxylase; EC 1.14.16.2; TYH; Dystonia 14; EC 1.14.16; DYT14; and DYT5b. External Ids for Th are HGNC: 11782; Entrez Gene: 7054; Ensembl: ENSG00000180176; OMIM: 191290 and UniProtKB: P07101.

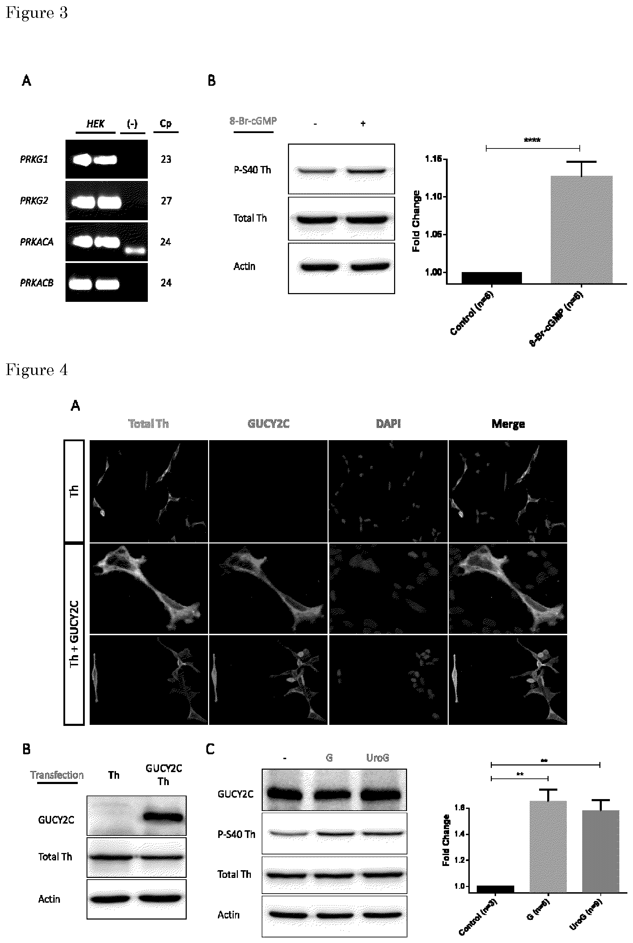

With the term "modulating dopamine production" is meant the up or down adjustment of a dopamine level. The production is done by a dopaminergic cell. Modulating the production thus means up or down adjustment of a dopamine level in a dopaminergic neuron, typically with the aim to increase dopamine release from the dopaminergic cell. The release is preferably in the projection area of the dopaminergic midbrain neurons, preferably of the substantia nigra. The projection area is preferably the striatum. Modulation of dopamine production is achieved by modulating signaling by GUCY2C in the dopaminergic cell. The modulation is preferably an up adjustment of the signaling, i.e. an up adjustment of the level of cGMP in the dopaminergic cell. The modulation is typically achieved by modulating tyrosine hydroxylase (Th) activity in the cell. Th can be measured in various ways. An obvious way is to measure the production of the Th enzymatic product in the context of the substrate provided to it. Th activity has been found to be dependent on serine phosphorylation. In the present invention a method has been developed to modulate dopamine production by modulation Th Ser40 phosphorylation. As indicated herein dopamine production by a dopaminergic cell is increased by increasing the level of Ser40 phosphor Th (P-S40 Th) in the cell, measured in relation to the total level of Th in the cell. Typically the level of Ser40 phosphor is compared to the level of Th that is not phosphorylated on Ser40. This is typically done in an assay as described in the examples in relation to figures 3-5.

The invention also provides a method of modulating the level of phosphorylation of Ser40 of tyrosine hydroxylase in a dopaminergic cell, the method comprising modulating signaling by GUCY2C in said cell. Up or down adjustment of the Ser40 phosphorylation is typically an increase of the level of P-S40 Th of at least 10%, 20%, 30%, preferably at least 50% and more preferably at least 80% when compared to the level in the unadjusted cell under otherwise identical circumstances. In case of up adjustment the P-S40 Th level is preferably adjusted to at least 100% and preferably at least 200% more than the unadjusted level.

Up adjustment of the dopamine level is typically a respective increase of the dopamine level of at least 10%, 20%, 30%, preferably at least 50% and more preferably at least 80% when compared to the level in the unadjusted cell under otherwise identical circumstances. The dopamine level is preferably adjusted to at least 100% and preferably at least 200% more than the unadjusted level.

Up adjustment of the cGMP level is typically a respective increase of the cGMP level of at least 10%, 20%, 30%, preferably at least 50% and more preferably at least 80% when compared to the level in the unadjusted cell under otherwise identical circumstances. The cGMP level is preferably adjusted to at least 100% and preferably at least 200% more than the unadjusted level.

In a preferred embodiment the invention provides a method of modulating dopamine production by a dopaminergic cell, the method comprising modulating signaling by Guanylate Cyclase 2C (GUCY2C) in said cell. In a preferred embodiment the invention provides a method of increasing dopamine production by a dopaminergic cell, the method comprising increasing signaling by Guanylate Cyclase 2C (GUCY2C) in said cell.

The invention also provides a method of increasing the level of phosphorylation of Ser40 of tyrosine hydroxylase in a dopaminergic cell, the method comprising increasing signaling by GUCY2C in said cell.

An increase in the level of dopamine is typically an increase of the dopamine level of at least 10%, 20%, 30%, preferably at least 50% and more preferably at least 80% when compared to the level in the unadjusted cell under otherwise identical circumstances. The dopamine level is preferably increased to at least 100% and preferably at least 200% more than the unadjusted level.

A method of increasing dopamine production by a dopaminergic cell, is preferably achieved by increasing signaling by Guanylate Cyclase 2C (GUCY2C) in said cell. The cGMP level is typically increased by at least 10%, 20%, 30%, preferably at least 50% and more preferably at least 80% when compared to the level in the unadjusted cell under otherwise identical circumstances. Preferably increased to at least 100% and preferably at least 200% more than the unadjusted level.

Signaling of GUCY2C can be modulated in various ways. One way is to modulate the level of mRNA that codes for GUCY2C. This can be done by introducing an expression cassette with a GUCY2C coding region into the cell. A higher level of GUCY2C mRNA in the cell leads to more GUCY2C in the membrane and an increased signaling of GUCY2C in the cell.

Up adjustment of the level of GUCY2C signaling is preferably achieved by providing a GUCY2C containing cell with a GUCY2C agonist. The agonist is preferably a member of the guanylin family of the peptides, preferably guanylin, uroguanylin or a functional derivative thereof.

The method of modulating and preferably increasing dopamine production preferably further comprises providing the dopaminergic cell with a PDE inhibitor. In some embodiments the PDE inhibitor is a non-selective inhibitor such as IBMX.

Up adjustment of the level P-S40 Th is preferably achieved by providing a GUCY2C containing cell with a GUCY2C ligand, preferably an agonist The agonist is preferably a member of the guanylin family of the peptides, preferably guanylin, lymphoguanylin, uroguanylin or a functional derivative thereof. Up adjustment of the level of GUCY2C signaling can also be achieved by increasing the number of GUCY2C receptors on the cell membrane. One method is by introducing an expression cassette comprising a GUCY2C coding region into the dopaminergic cell. The expression cassette is preferably introduced by means of a viral vector. Nonlimiting examples of suitable vectors are adeno-associated virus vectors or lentivirus vectors.

The method of modulating and preferably increasing the level of P-S40 Th preferably further comprises providing the dopaminergic cell with a PDE inhibitor. In some embodiments the PDE inhibitor is a non-selective inhibitor such as IBMX.

The invention provides a method of determining whether a compound is capable modulating dopamine production by a dopaminergic cell comprising determining whether a compound is capable of modulating the activity of a GUCY2C gene, an RNA or a protein encoded by the gene. The method preferably further comprises contacting the dopaminergic cell with the compound; and determining whether the compound is capable of modulating the production of dopamine by the dopaminergic cell. The invention further provides a use of a GUCY2C gene, an RNA or a protein encoded by the gene as a target for identifying a compound that is active in modulating tyrosine hydroxylase activity a dopaminergic cell. With the new use of modulating GUCY2C activity in a cell, the artisan will appreciate that it is possible to find new GUCY2C agonists.

The invention provides a GUCY2C agonist for use in the treatment of an individual that has Parkinson's disease. The use preferably further comprises administering a PDE inhibitor. The PDE inhibitor can be a non-selective PDE-inhibitor. The individual preferably has Parkinson's disease stage 1, 2, 3 or 4. In a preferred embodiment the individual has Parkinson's disease stage 1, 2 or 3, preferably 1 or 2 and preferably stage 1. It was noted in the present invention that a guanylin agonist stimulates the production of dopamine by relevant cells in a physiological way and has much less side effects that a treatment with levodopa. The treatment is particularly effective in early stages of the disease, where the limitation of dopamine production in the individual just becomes apparent in the expression of mild symptoms. In such cases the individual does not have enough dopamine production typically due to the fact to the partial disappearance of dopaminergic cells. Particularly in the early stages, however, the individual typically has ample cells to provide adequate production of dopamine upon the treatment of the invention. Treatment can be successful to revert the individual to symptomless. The agonist is preferably guanylin or a functional derivative thereof. The treatment preferably further comprises a administering a PDE inhibitor to the individual.

The GUCY2C agonist is active on its own. Thus the invention provides a pharmaceutical composition that comprises a GUCY2C agonist and a pharmaceutically acceptable excipient, wherein the GUCY2C agonist is the only pharmaceutically active ingredient for the treatment of Parkinson's disease. WO2013/016662 describes a GUCY2C ligand conjugate to a payload for the treatment of Parkinson's disease. The inventors of WO2013/016662 failed to recognize the effect of a GUCY2C agonist on the production of dopamine and the treatment of individuals with Parkinson's disease, particularly early stage disease. WO2013/016662 describes that it is the payload that has the therapeutic effect. According the WO2013/016662 the payload can be a therapeutic or diagnostic moiety. In the present invention the agonist is provided without such an additional moiety. It is provided without a diagnostic or therapeutic moiety as described in WO2013/016662. The therapeutic effect of a GUCY2C agonist alone remained hidden in WO2013/016662. A treatment of Parkinson's disease as described herein preferably further comprising providing said cell with a PDE inhibitor. In some embodiments the PDE inhibitor is a non-selective inhibitor such as IBMX. In this embodiment the GUCY2C agonist is without an additional payload and without a label for diagnostic or detection purposes. The GUCY2C agonist and the PDE inhibitor are preferably the only pharmaceutically active ingredients of the medicament for the treatment of Parkinson's disease.

The invention also provides a method of increasing dopamine production by a dopaminergic cell, the method comprising increasing signaling by Guanylate Cyclase 2C (GUCY2C) in said cell. Further provided is a method of increasing the level of phosphorylation of Ser40 of tyrosine hydroxylase in a dopaminergic cell, the method comprising increasing signaling by GUCY2C in said cell. The signaling by GUCY2C is preferably increased by contacting said cell with a GUCY2C agonist. Said agonist is preferably guanylin or a functional derivative thereof. The method preferably further comprising providing said cell with a PDE inhibitor. Said cell is preferably a dopaminergic neuron, preferably a midbrain dopaminergic neuron.

In the examples the invention describes a test system for determining whether a compound, preferably a GUCY2C ligand, preferably a GUCY2C agonist, is capable of activating GUCY2C, or increasing the level of ser40 phosphorylation of tyrosine hydroxylase, or increasing dopamine production. Such a test system can suitably comprise an isolated or recombinant human cell comprising ectopic expression of human GUCY2C and/or ectopic expression of human tyrosine hydroxylase. Such a cell is ideally suited to perform methods to identify GUCY2C agonists with similar or better properties than a natural GUCY2C agonist. In a preferred embodiment the cell comprises ectopic expression of human GUCY2C and ectopic expression of human tyrosine hydroxylase. Ectopic expression is preferred because such cells can be chosen for better performance in *in vitro* screening systems. Nerve cells that typically express Th are often not the best performing cells and if suitable for robust, easy and quick *in vitro* cultures, have lost or gained properties that hinder analyses of the results. Ectopic expression systems also allow the easy identification/confirmation of the pathway by which the compound works as controls are easily produced (same cells without the ectopic expression of one or more of the proteins). With the term "ectopic expression" is meant the expression of a gene or the presence of a protein in a tissue or cell where it is not normally expressed. Various cells can be used in this context. It is preferably an immortalized cell. Preferably a cell adapted to *in vitro* culture. In a preferred embodiment the cell is a primate cell, preferably a human cell. In a particularly preferred embodiment the cell is a HEK cell. The mentioned cells are particularly suited for identifying a candidate compound for modifying dopamine production by a dopaminergic cell. The invention thus also provides a method for identifying a candidate compound for modifying dopamine production by a dopaminergic cell, the method comprising culturing a cell as mentioned in this paragraph; contacting said cell with the candidate compound and determining the activity of tyrosine hydroxylase in said cell. The activity of Th can be measured in various ways. In a preferred embodiment the activity is measured by measuring the phosphorylation of Th, preferably the phosphorylation of ser40 of tyrosine hydroxylase is determined.

The invention also provides the use of a GUCY2C gene, an RNA or a protein encoded by the gene as a target for identifying a compound that is active in modulating tyrosine hydroxylase activity in a dopaminergic cell. Further provided is a method of treatment of an individual that has Parkinson's disease, or is at risk of developing the disease, the method comprising administering a GUCY2C agonist to the individual in need thereof. In a preferred embodiment the method further comprises administering a PDE inhibitor to the individual.

The PDE inhibitor in the context of embodiments of present invention can be a non-selective PDE inhibitor such as IBMX.

The individual that has Parkinson's disease, or is at risk of developing the disease is preferably a human. The GUCY2C protein and/or gene is preferably a human GUCY2C protein or gene. The member of the guanylin family of peptides is preferably a human member. Guanylin or uroguanylin is preferably human guanylin or uroguanylin. A functional derivative of guanylin or uroguanylin is preferably a functional derivative of human guanylin or uroguanylin.

The ligand such as an agonist, the PDE inhibitor or the combination thereof are preferably administered to the brain of the individual. In some embodiments the PDE inhibitor is a non-selective inhibitor such as IBMX.

Parkinson's Disease typically start-off slowly with minor symptoms. The disease can progress slowly or fast. During this progression the symptoms grow worse and new symptoms can become apparent. Various stages of disease can be identified that correlate more or less with the progressive loss of dopaminergic neurons from the substantia nigra. Often 5 stages are identified. Stage 1 is typically characterized by mild symptoms that generally do not interfere with daily activities. Tremor and other movement symptoms typically occur on one side of the body only. Friends and family may notice changes in posture, walking and facial expressions. In stage 2 the symptoms start getting worse. Tremor, rigidity and other movement symptoms affect both sides of the body. Walking problems and poor posture may become apparent. In this stage, the person is still able to live alone, but completing day-to-day tasks becomes more difficult and may take longer. Stage 3 is considered mid-stage in the progression of the disease. Loss of balance and slowness of movements are hallmarks of this phase. Falls are more common. Though the person is still fully independent, symptoms significantly impair activities of daily living such as getting dressed and eating. Parkinson's, symptoms are severe and very limiting in stage 4. It's possible to stand without assistance, but movement may require a walker. The person needs help with activities of daily living and is unable to live alone. Stage 5 is the most advanced and debilitating stage of Parkinson's disease. Stiffness in the legs may make it impossible to stand or walk. The person requires a wheelchair or is bedridden. Around-the-clock nursing care is required for all activities. The person may experience hallucinations and delusions. While stage five focuses on motor symptoms, the Parkinson's community acknowledges that there are many important non-motor symptoms as well.

For medical uses in the context of Parkinson's and the treatment of individuals that have Parkinson's disease it is preferred that the individual does not have a completely advanced form of Parkinson's disease. In such cases the substantia nigra is (almost) completely destroyed. For the present invention it is preferred that the individual has Parkinson's disease stage 1, 2, 3 or 4. In a preferred embodiment the individual has Parkinson's disease stage 1, 2, or 3. Preferably stage 1 or stage 2. An advantage of the present invention is that early stages of the disease can be treated adequately without inducing many of the side-effects associated with the present standard therapies. Dopaminergic neurons of the substantia nigra are stimulated to produce more dopamine to compensate for the loss of dopaminergic cells in the substantia nigra while at the same time not over-producing dopamine, or having excessive dopamine production in non-target cells, i.e. dopaminergic neurons that are not in the substantia nigra.

Preferably, the compounds of the invention are used as a medicine. Medicines of the invention can suitably be used for the treatment of an individual that has Parkinson's disease or is at risk of having Parkinson's disease. Pharmaceutical compositions of the invention are particularly suited for increasing the production of dopamine by dopaminergic cells as described herein.

### Delivery Methods

Once formulated, a compound or composition of the invention can be administered directly to a subject; or delivered to cells in vitro.

Direct delivery of the compositions to a subject will generally be accomplished by injection, either subcutaneously, intraperitoneally, intravenously or intramuscularly, or delivered to the interstitial space of a tissue. Other modes of administration include topical, oral, catheterized and pulmonary administration, suppositories, and transdermal applications, needles, and particle guns or hyposprays. Dosage treatment may be a single dose schedule or a multiple dose schedule. Delivery is preferably to the brain of the individual. A preferred route of administration is via the epithelium of the nose.

Generally, delivery of nucleic acids for both ex vivo and in vitro applications can be accomplished by, for example, gene transfer using a vector including a virus, dextran-mediated transfection, calcium phosphate precipitation, polybrene^{®} mediated transfection, protoplast fusion, electroporation, encapsulation of the polynucleotide(s) in liposomes, and direct microinjection of the DNA into nuclei, all well known in the art.

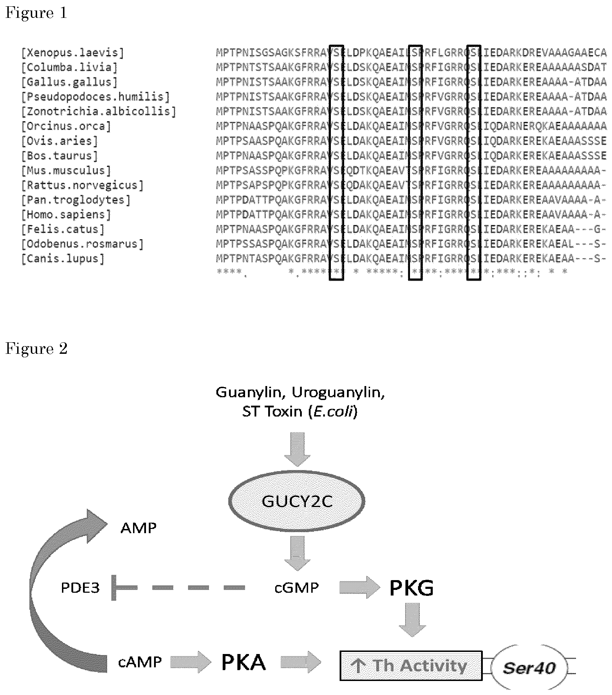

Without being bound by theory it is believed that modulation and preferably increasing the signaling of GUCY2C in a dopaminergic cell of an individual that has Parkinson's disease or is at risk of developing the disease modulates and preferably increases Th activity in the cell. The treatment alleviates motor abnormalities in individual with PD or at risk thereof. This enzyme, by catalyzing the rate-limiting step in dopamine production, sets the overall speed of the whole biosynthetic pathway. Th has an N-terminal regulatory domain, a central catalytic domain and a C-terminal tetramerization domain (Tekin I et al., 2014). The most appealing domain to increase Th activity is located in the N-terminal region, which includes three serines susceptible to phosphorylation (i.e. Ser19, Ser31 and Ser40). The regulatory importance of these residues is reflected in their broad evolutionary conservation (Fig.1). Independent phosphorylation of Ser31 and Ser40 is known to increase Th activity in vitro and in situ. Ser31 phosphorylation exclusively operates by raising the affinity of Th for one of its cofactors (i.e. BH4), whereas Ser40 also impedes the negative feedback loop by blocking the interaction of dopamine with the catalytic domain of Th (Dunkley PR et al., 2004). Ser40 is thus a promising target, and we believe that boosting its phosphorylation solves an important limitation of levodopa treatment.

It is an object of the invention to increase Th activity by inducing Ser40 phosphorylation specifically in the midbrain. The main kinases known to phosphorylate this residue are cAMP- and cGMP-dependent protein kinases (PKA and PKG, respectively) (Campbell DG et al., 1986; Roskoski R Jr et al., 1987). Both enzymes are basally inactive due to the interaction of the regulatory region with the catalytic center. While the regulatory and catalytic counterparts of PKA correspond to separate polypeptides, PKG is a single amino acidic chain including both domains. In order to be activated, both kinases depend on the interaction of the corresponding cyclic nucleotide with the regulatory region of the enzyme, so that the catalytic center is released to phosphorylate different substrates (Scholten A et al., 2008). Among their various targets, Ser40 in Th is the preferred target in the present invention.

Particulate guanylyl cyclase 2C (GUCY2C) in the brain was reported by Gong R et al., 2011. An identical observation is registered in the Allen Brain Atlas. GUCY2C expression is restricted to A8-A10 neurons. Until less than a decade ago, this receptor was thought to be exclusively expressed in the gastrointestinal tract, where it interacts with endogenous ligands such as guanylin, uroguanylin or the heat-stable toxin secreted by E.coli (ST Toxin). Upon activation, this receptor produces cGMP and ultimately decreases water absorption by stimulating an outward-chlorine channel (CFTR) and inhibiting an inward-sodium channel (NHE3) (Fiskerstrand T et al., 2012).

The present invention shows that GUCY2C activation increases Ser40 phosphorylation and thus Th activity specifically in the midbrain. Without being bound by theory it is believed that this is achieved by two mechanisms. Production of cGMP activates PKG and indirectly activate PKA by the inhibition of phosphodiesterase 3, an enzyme involved in the breakdown of cAMP to its non-cyclic form (Fig. 2) (Fiskerstrand T et al., 2012).

The present approach addresses a problem of specificity in the brain, as it affects the nigrostriatal pathway and maintains the ability to induce dopamine synthesis and its ultimate release. Th is co-expressed with GUCY2C in the midbrain, and GUCY2C knock-out mice exhibit a lower dopamine release in the striatum as compared to their wildtype (wt) littermates (Gong R et al., 2011).

For the purpose of clarity and a concise description features are described herein as part of the same or separate embodiments, however, it will be appreciated that the scope of the invention may include embodiments having combinations of all or some of the features described.

### Brief description of the drawings

**Figure 1****.** Multiple sequence alignment of Th-regulatory domain across different species. The three highlighted serine residues (Ser19, Ser31 and Ser40) are susceptible to phosphorylation and are well conserved throughout evolution. The phosphorylation of Ser31 and Ser40 directly correlates with an increased activity of the enzyme. Note that the amino acids surrounding both residues are virtually unchanged, thereby conserving the consensus sequence and explaining why those serines are phosphorylated in different organisms. '*' represents maximal conservation, followed by ':'and '.'.
**Figure 2****.** Theoretical pathways by which GUCY2C activation can lead to an increased Th activity. One alternative is the direct activation of PKG by cGMP, while the other consists in the indirect activation of PKA via the cGMP-mediated inhibition of PDE3, an enzyme involved in degrading cAMP into its non-cyclic form.
**Figure 3****.** HEK cells are a suitable model to study GUCY2C-induced Ser40 phosphorylation. (A) RT-qPCR data from intact HEK extracts. Input material was 10 ng of total RNA. Cp corresponds to the cycle number in which the expression starts to be considered positive. (-) represents the water control, which is negative in every case. (B) HEK cultures were transfected with Th and treated with a cGMP analogue 48 hours post-plating. Changes in Ser40 phosphorylation were studied. The graph represents the statistical analysis (n=6; Mean±SEM). Unpaired Student's t-test (p-value: ****<0.0001). Hereon, 'Fold Change' corresponds to the ratio P-Ser40/Total Th.
**Figure 4****.** GUCY2C activation increases Ser40 phosphorylation. Immunocytochemistry images (A) and western blotting analysis (B) of HEK cultures transfected with Th or Th+GUCY2C. (C) Co-transfected HEK cells were treated with guanylin (G) or uroguanylin (UroG) and changes in Ser40 phosphorylation were studied. The graph represents the statistical analysis (n=3 for Control, n=6 for Guanylin, n=9 for Uroguanylin; Mean±SEM). Unpaired Student's t-test (p-value: **<0.01).

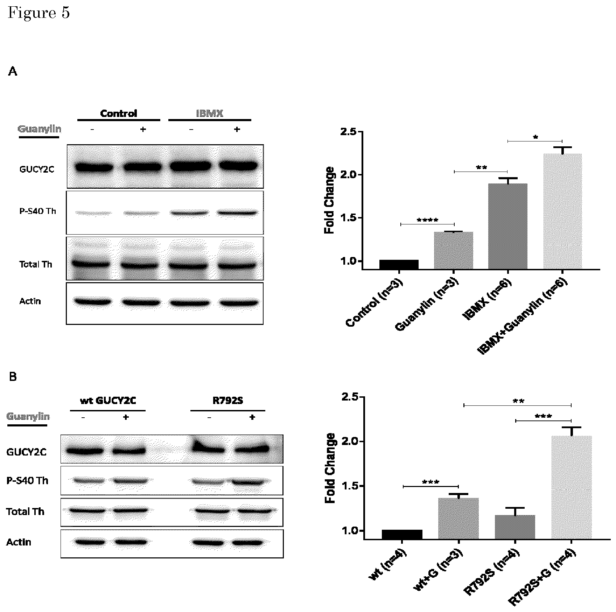

**Figure 5****.** The increase in Ser40 phosphorylation is proportional to the produced amount of cGMP upon GUCY2C stimulation. (A) HEK cultures were treated with guanylin, in the presence or absence of IBMX. The graph shows the statistical analysis (n=3 for Control±Guanylin, n=6 for IBMX±Guanylin; Mean±SEM). Unpaired Student's t-test (p-values: ****<0.0001, **<0.01, *<0.05). (B) HEK cells were co-transfected with Th and either the wt GUCY2C or a gain-of-function mutant (R792S). Ser40 phosphorylation was studied following guanylin treatment (G). The graph shows the statistical analysis (n=4 for wt, n=3 for wt+Guanylin, n=4 for R792S±Guanylin; Mean±SEM). Unpaired Student's t-test (p-values: ***<0.001, **<0.01).

### Examples

### Materials and Methods

### Cell culture

HEK cells were maintained in 100-mm Petri dishes and grown in DMEM medium supplemented with L-glutamine, penicillin-streptomycin (Pen&Strep) and 10% heat-inactivated fetal bovine serum (HIFBS). Growing conditions were 37°C and 5% CO2. For every-other-day passages, HEK cultures were split at a 1:3 dilution From Friday to Monday, the split proportion was doubled for all cell lines. For passaging, cultures were rinsed with PBS and incubated with 1 mL of trypsin during 5 minutes. Cells were resuspended in growth medium and finally split to the proper dilution. The experiments were performed in 12-well plates. For immunocytochemistry analysis, sterile 18-mm coverslips were added prior to the plating. 500 µL of cell resuspension was seeded in each well. If experiments were to be performed 48 hours after the plate preparation, 1:5 was the seeding ratio for HEK cells. When the experiments took place 96 hours post-plating, 1:7 was the working dilution for HEK cells. Unless otherwise stated, HEK cultures were processed 96 hours post-plating.

### Cell transfection

Plasmids encoding mouse Th and human GUCY2C had a pcDNA3.1 backbone. HEK cells were transfected using the calcium-phosphate method. 12-well plates were refreshed with 500 µL of growth medium before transfection. For each pair of wells, plasmids of interest did not exceed 2.5 µg and were filled up to 5 µg with the empty construct pBlueScript. The plasmid mixture was taken into a final concentration of 250 mM CaCl2 in a total volume of 110 µL. A complementary tube was filled with 110 µL of HEPES-buffered saline 2X (HEBS 2X: 1.5 mM Na2HPO4, 50 mM HEPES pH 7.05, 280 mM NaCl). The tube with CaCl2 was pipetted dropwise into the HEBS and mixed gently. After 60 seconds of incubation, 110 µL of the final mixture was added to each well. The medium was replaced within the next 24 hours.

### Chemical treatment

24 hours prior to the administration of the different compounds, cells were serum-starved with DMEM medium supplemented with L-glutamine and Pen&Strep.. Unless otherwise stated, the concentration of the different reagents is reported in Table 1 and the duration of the treatment was 1 hour.

### Western blotting

Cells were harvested in 150 µL of Laemmli sample buffer (2% SDS, 10% glycerol, 60 mM Tris-Cl pH 6.8, 0.01% bromophenol blue, 50 mM freshly added DTT). Wells were duplicated in pairs and pooled into the same tubes to minimize variability. Samples were sonicated at maximum potency during 3 minutes, heated at 95°C for 5 minutes and spun down briefly before loading. Running gels were 10% polyacrylamide except for the GUCY2C detection, which was optimal in 7% gels (375 mM Tris-Cl pH 8.8, 0.1% APS, 0.1% SDS, 0.04% TEMED). Stacking gels were 5% polyacrylamide (125 mM Tris-Cl pH 6.8, 0.1% APS, 0.1% SDS, 0.04% TEMED).

Up to 35 µL of sample was loaded in each slot and run in the presence of tris-glycine buffer and 0.1% SDS. Running conditions were 100 V during the first 20 minutes, followed by 160 V until the migration front reached the bottom of the gel. The transference was performed at 100 V onto 0.2 µm nitrocellulose membranes, in the presence of tris-glycine buffer and 20% methanol. The blotting duration was 140 minutes except for the detection of GUCY2C, which was transferred during 240 minutes in the presence of 0.1% SDS. Membranes were then submerged in Ponceau S solution to check blotting efficiency (0.1% Ponceau S, 5% acetic acid). After several washings with DEMI water, blots were incubated during 1 hour in the presence of 5% milk powder and TBS-T (154 mM NaCl, 49.5 mM Tris-Cl pH 7.4, 0.1% Tween-20). The incubation with the primary antibodies was performed O/N at 4°C in TBS-T (consult Table 2 for dilution and species). Membranes were rinsed in TBS-T during 1 hour to remove the excess antibody. The incubation with the secondary antibodies took place during 60 minutes at room temperature (1:10,000 dilution in TBS-T, with the exception of the goat secondary which was diluted 1:20,000). Secondary antibodies were fused to the horseradish peroxidase and raised against the host species of the primary antibody. After additional washings during 1 hour, blots were exposed to an enhanced chemiluminescence solution and the signal was detected using an Odyssey imager (LI-COR). Band densitometry was performed with LI-COR Image Studio Lite. For graph quantifications, 'Fold Change' represents the ratio P-Ser40/Total Th. Statistical comparisons between pair of groups correspond to unpaired Student's t-tests, as calculated with GraphPad Prism. Asterisks denote the following p-values: *<0.05, **<0.01, ***<0.001, ****<0.0001.

### Immunocytochemistry

The growth medium was removed from the 12-well plates containing 18-mm coverslips. Following a washing step with ice-cold PBS, cells were fixed in 4% paraformaldehyde during 20 minutes and subsequently washed 3 times with PBS (137 mM NaCl, 2 mM KH2PO4, 100 mM Na2HPO4, 2.7 mM KCl). The blocking was performed during 1 hour in the presence of 4% fetal donkey serum and 0.2% Triton X-100. Coverslips were then incubated O/N at 4°C with the primary antibodies, diluted in PBS and 0.2% Triton X-100 (see Table 2 for antibody information). After rinsing the cells 3 times with PBS, the incubation with the secondary antibody took place during 2 hours at room temperature (1:1,000 dilution in PBS). An additional washing step with PBS preceded the 5-minute incubation with DAPI, diluted 1:3,000 in PBS. After a final rinse with PBS, coverslips were embedded with Fluorosave onto 60x20-mm slides. Final preparations were allowed to harden O/N at 4°C before performing the analysis under the fluorescence microscope (Leica).

### RNA isolation and RT-qPCR

The growth medium was discarded from the culture dish and 1 mL of Trizol was added to each well. Cells were directly lysed in the plate and harvested into Eppendorf tubes. 200 µL of chloroform was added and the mixture was incubated during 3 minutes after intense shaking. Samples were centrifuged at 4°C during 15 minutes at 12,000 rcf. The upper aqueous phase was then collected into a new tube and the lower phases were disposed of. 10 µg of glycogen and 500 µL of isopropanol were added to the preparations. After a 10-minute incubation, samples were centrifuged at 4°C during 10 minutes at 12,000 rcf. The supernatant was cautiously removed from the tubes and the pellet was subsequently rinsed with 1 mL of 75% ethanol. A brief vortex step was followed by a centrifugation at 4°C during 5 minutes at 12,000 rcf. Supernatants were discarded and, once the pellets were moderately dry, resuspension took place in 30 µL of RNAse-free water. Regarding the RT-qPCR analysis, purified RNA samples were diluted to a final concentration of 2.6 ng/µL. For each reaction well, volumes of the different reagents were the following: 5 µL SYBR Green buffer 2X, 0.1 µL reverse transcriptase, 0.1 µL RNAse inhibitor, 0.5 µL forward primer 10 µM, 0.5 µL reverse primer 10 µM, 3.8 µL purified RNA 2.6 ng/µL. Reactions were carried out in a LightCycler 480 (Roche) according to the QuantiTect SYBR Green RT-PCR handbook (Quiagen). The ribosomal RNA 18S was used as loading control. Primers were designed using Primer-BLAST and their specificity was tested in a 1.5% agarose gel once the RT-qPCR was terminated.

### Results

### HEK cells can be employed to study the induction of Ser40 phosphorylation upon GUCY2C activation

We studied the regulation of Th via GUCY2C activation in vitro. Among the various cell lines which are available, we firstly employed HEK cells since they are easily transfectable and had been previously used to study the role of GUCY2C in cGMP production (Fiskerstrand T et al., 2012; Müller T et al., 2015). We checked the presence of the mRNAs encoding the kinases. RT-qPCR data showed a positive expression of the different isoforms of PKG (PRKG1 and PRKG2) and the catalytic centers of PKA (PRKACA and PRKACB), suggesting that HEK cells are a suitable model for our purposes (Fig.3A). However, neither TH nor GUCY2C are endogenously expressed in this cell line. With the aim of checking if the cGMP-derived signaling could promote Ser40 phosphorylation, we transfected HEK cells with a Th-encoding plasmid and treated them with an artificial cGMP analogue (i.e. 8-Bromo-cGMP). As expected, we detected a small but significant increase in P-Ser40 levels upon the compound administration (Fig.3B).

Next, in order to study the potential effects of GUCY2C activation on Th phosphorylation, we performed co-transfections with the plasmids encoding both proteins. Western blotting analysis revealed HEK cultures expressing Th and GUCY2C. Sole transfection with Th served as control for GUCY2C expression and recognition (Fig.4B). Immunocytochemistry analysis allowed us to identify individual cells co-expressing both proteins, essential requirement to study the potential link between the receptor and Ser40 phosphorylation (Fig.4A). Most importantly, within this experimental paradigm in which the kinases are endogenously expressed while Th and GUCY2C are co-transfected, we were able to increase P-Ser40 levels upon separate administration of different receptor ligands (i.e. guanylin and uroguanylin) (Fig.4C).

### The induction of P-Ser40 upon GUCY2C activation is proportional to the levels of cGMP

Regarding the two ways by which the receptor activation might induce Ser40 phosphorylation, both depend on the initial production of cGMP (Fiskerstrand T et al., 2012). To test this assumption, we combined guanylin with the administration of IBMX, a general inhibitor of phosphodiesterases (PDEs). This family of enzymes breaks down cyclic nucleotides to their non-cyclic forms (Bender AT and Beavo JA, 2006). Therefore, as the cyclic variants can activate PKA and PKG, we hypothesized that inhibiting PDEs would potentiate the induction of P-Ser40 upon GUCY2C stimulation. Surprisingly, treatment with IBMX potentiated the increase in Ser40 phosphorylation as compared to guanylin administration (Fig.5A). These data show that the basal pool of cyclic nucleotides in HEK cells is large and continuously subjected to degradation by PDEs as a negative-feedback mechanism. The combined treatment of guanylin with IBMX further increased Ser40 phosphorylation in relation to IBMX alone, suggesting that the basal and the GUCY2C-derived pools of cyclic nucleotides display additive effects (Fig.5A).

However, since IBMX is a general PDE inhibitor, its effects on Ser40 phosphorylation result from the increase in the levels of both cAMP and cGMP. To correlate the extent of Ser40 phosphorylation exclusively with the amount of cGMP produced by the receptor, we devised an alternative approach. A previous paper described the existence of human naturally-occurring mutations in the GUCY2C-encoding gene, rendering a set of gain-of-function receptors (Müller T et al., 2015). These variants produce more cGMP than the wt form in the presence of guanylin. The mutant with the highest activity contains an arginine to serine substitution in the position 792 (R792S), located at the starting point of the catalytic domain.

HEK cultures were co-transfected with the plasmids encoding Th and either the wt or the R792S variant of GUCY2C. When compared to the wt receptor, the boost in Ser40 phosphorylation upon guanylin treatment was 1.5-fold higher when the R792S mutant was co-transfected (Fig.5B).

The present invention shows methods to increase Th activity specifically in the nigrostriatal pathway. We focused on the phosphorylation of its regulatory domain. Both Ser31 and Ser40 phosphorylation have been reported to promote such an effect (Dunkley PR et al., 2004).

In conclusion, our experiments indicate that the signaling pathway underlying GUCY2C activation is specific for each model system. HEK cells show an induction of Ser40 phosphorylation which is proportional to GUCY2C activity levels.

The present invention shows that the combination of GUCY2C ligands with PDE inhibitors serve to increase dopamine production by dopaminergic cells. This finding is used as a therapy for PD.

Current levodopa treatments are often supplemented with AADC inhibitors which cannot reach the brain but prevent dopamine synthesis in peripheral tissues (Ahlskog JE et al., 1989). The therapy the invention provides provides a specific component, given the selective brain expression of GUCY2C in the SNpc, and a more or less non-selective component provided by the wide expression of PDE.. An unspecific inhibition of PDEs is not a major complication for clinical treatments. In fact, some widely-used drugs are inhibitors of PDEs which are expressed in various off-target tissues. A representative example is sildenafil (Viagra), a PDE5-selective inhibitor. This phosphodiesterase is expressed in the penis, where it is intended to exert its effects, but also in the heart, pancreas, kidney or cerebellum (Lin CS, 2004).

### Cited art

- Ahlskog JE, Tyce GM, Kelly PJ, van Heerden JA, Stoddard SL, Carmichael SW (1989). Cerebrospinal fluid indices of blood-brain barrier permeability following adrenal-brain transplantation in patients with Parkinson's disease. Exp Neurol. 1989 Aug;105(2):152-61.
- Allen Brain Atlas (http://mouse.brain-map.org/).
- Baladron J, Hamker FH (2015). A spiking neural network based on the basal ganglia functional anatomy. Neural Netw. 2015 Jul;67:1-13. doi: 10.1016/j.neunet.2015.03.002. Epub 2015 Mar 24.
- Bender AT, Beavo JA (2006). Cyclic nucleotide phosphodiesterases: molecular regulation to clinical use. Pharmacol Rev. 2006 Sep;58(3):488-520.
- Boess FG, Hendrix M, van der Staay FJ, Erb C, Schreiber R, van Staveren W, de Vente J, Prickaerts J, Blokland A, Koenig G (2004). Inhibition of phosphodiesterase 2 increases neuronal cGMP, synaptic plasticity and memory performance. Neuropharmacology. 2004 Dec;47(7):1081-92.
- Campbell DG, Hardie DG, Vulliet PR (1986). Identification of four phosphorylation sites in the N-terminal region of tyrosine hydroxylase. J Biol Chem. 1986 Aug 15;261(23):10489-92.
- Carta M, Carlsson T, Kirik D, Björklund A (2007). Dopamine released from 5-HT terminals is the cause of L-DOPA-induced dyskinesia in parkinsonian rats. Brain. 2007 Jul; 130(Pt 7):1819-33. Epub 2007 Apr 23.
- Clarke CE (2004). Neuroprotection and pharmacotherapy for motor symptoms in Parkinson's disease. Lancet Neurol. 2004 Aug;3(8):466-74.
- Dale Purves, George J Augustine, David Fitzpatrick, Lawrence C Katz, Anthony-Samuel LaMantia, James O McNamara, and S Mark Williams (2001). Neuroscience, 2nd edition. ISBN-10: 0-87893-742-0.
- Defer N, Best-Belpomme M, Hanoune J (2000). Tissue specificity and physiological relevance of various isoforms of adenylyl cyclase. Am J Physiol Renal Physiol. 2000 Sep;279(3):F400-16.
- Dunkley PR, Bobrovskaya L, Graham ME, von Nagy-Felsobuki EI, Dickson PW (2004). Tyrosine hydroxylase phosphorylation: regulation and consequences. J Neurochem. 2004 Dec;91(5):1025-43.
- Fiskerstrand T, Arshad N, Haukanes BI, Tronstad RR, Pham KD, Johansson S, Håvik B, Tønder SL, Levy SE, Brackman D, Boman H, Biswas KH, Apold J, Hovdenak N, Visweswariah SS, Knappskog PM (2012). Familial diarrhea syndrome caused by an activating GUCY2C mutation. N Engl J Med. 2012 Apr 26;366(17):1586-95. doi: 10.1056/NEJMoa1110132. Epub 2012 Mar 21.
- Gershanik OS (2015). Improving L-dopa therapy: the development of enzyme inhibitors. Mov Disord. 2015 Jan;30(1):103-13. doi: 10.1002/mds.26050. Epub 2014 Oct 21.
- Gong R, Ding C, Hu J, Lu Y, Liu F, Mann E, Xu F, Cohen MB, Luo M (2011). Role for the membrane receptor guanylyl cyclase-C in attention deficiency and hyperactive behavior. Science. 2011 Sep 16;333(6049):1642-6. doi: 10.1126/science.1207675. Epub 2011 Aug 11.
- Ikemoto S, Yang C, Tan A (2015). Basal ganglia circuit loops, dopamine and motivation: A review and enquiry. Behav Brain Res. 2015 Sep 1;290:17-31. doi: 10.1016/j.bbr.2015.04.018. Epub 2015 Apr 20.
- Jacobs FM, Smits SM, Noorlander CW, von Oerthel L, van der Linden AJ, Burbach JP, Smidt MP (2007). Retinoic acid counteracts developmental defects in the substantia nigra caused by Pitx3 deficiency. Development. 2007 Jul;134(14):2673-84.
- Joh TH, Park DH, Reis DJ (1978). Direct phosphorylation of brain tyrosine hydroxylase by cyclic AMP-dependent protein kinase: mechanism of enzyme activation. Proc Natl Acad Sci U S A. 1978 Oct; 75(10):4744-8.
- Kalia LV, Lang AE (2015). Parkinson's disease. Lancet. 2015 Aug 29;386(9996):896-912. doi: 10.1016/S0140-6736(14)61393-3. Epub 2015 Apr 19.
- Lin CS (2004). Tissue expression, distribution, and regulation of PDE5. Int J Impot Res. 2004 Jun;16 Suppl 1:S8-S10.
- Lochner A, Moolman JA (2006). The many faces of H89: a review. Cardiovasc Drug Rev. 2006 Fall-Winter;24(3-4):261-74.
- MacFarland RT, Zelus BD, Beavo JA (1991). High concentrations of a cGMP-stimulated phosphodiesterase mediate ANP-induced decreases in cAMP and steroidogenesis in adrenal glomerulosa cells. J Biol Chem. 1991 Jan 5;266(1):136-42.
- Mahlknecht P, Seppi K, Poewe W (2015). The Concept of Prodromal Parkinson's Disease. J Parkinsons Dis. 2015;5(4):681-97. doi: 10.3233/JPD-150685.
- Morgenroth VH 3rd, Hegstrand LR, Roth RH, Greengard P (1975). Evidence for involvement of protein kinase in the activation by adenosine 3':5'-monophosphate of brain tyrosine 3-monooxygenase. J Biol Chem. 1975 Mar 10;250(5):1946-8.
- Miiller T, Rasool I, Heinz-Erian P, Mildenberger E, Hülstrunk C, Miiller A, Michaud L, Koot BG, Ballauff A, Vodopiutz J, Rosipal S, Petersen BS, Franke A, Fuchs I, Witt H, Zoller H, Janecke AR, Visweswariah SS (2015). Congenital secretory diarrhoea caused by activating germline mutations in GUCY2C. Gut. 2016 Aug;65(8):1306-13. doi: 10.1136/gutjnl-2015-309441. Epub 2015 May 20.
- Navailles S, Milan L, Khalki H, Di Giovanni G, Lagière M, De Deurwaerdère P (2014). Noradrenergic terminals regulate L-DOPA-derived dopamine extracellular levels in a region-dependent manner in Parkinsonian rats. CNS Neurosci Ther. 2014 Jul;20(7):671-8. doi: 10.1111/cns.12275. Epub 2014 Apr 28.
- Nikolaev VO, Gambaryan S, Engelhardt S, Walter U, Lohse MJ (2005). Real-time monitoring of the PDE2 activity of live cells: hormone-stimulated cAMP hydrolysis is faster than hormone-stimulated cAMP synthesis. J Biol Chem. 2005 Jan 21;280(3):1716-9. Epub 2004 Nov 22.
- Nucleotide BLAST (https://blast.ncbi.nlm.nih.gov/Blast.cgi?PAGE_TYPE=BlastSearch).
- Postuma RB, Aarsland D, Barone P, Burn DJ, Hawkes CH, Oertel W, Ziemssen T (2012). Identifying prodromal Parkinson's disease: pre-motor disorders in Parkinson's disease. Mov Disord. 2012 Apr 15;27(5):617-26. doi: 10.1002/mds.24996.
- Primer BLAST (http://www.ncbi.nlm.nih.gov/tools/primer-blast/).
- Romi H, Cohen I, Landau D, Alkrinawi S, Yerushalmi B, Hershkovitz R, Newman-Heiman N, Cutting GR, Ofir R, Sivan S, Birk OS (2012). Meconium ileus caused by mutations in GUCY2C, encoding the CFTR-activating guanylate cyclase 2C. Am J Hum Genet. 2012 May 4;90(5):893-9. doi: 10.1016/j.ajhg.2012.03.022. Epub 2012 Apr 19.
- Roskoski R Jr, Vulliet PR, Glass DB (1987). Phosphorylation of tyrosine hydroxylase by cyclic GMP-dependent protein kinase. J Neurochem. 1987 Mar;48(3):840-5.
- Scansite3 (http://scansite3.mit.edu/#home).
- Scholten A, Aye TT, Heck AJ (2008). A multi-angular mass spectrometric view at cyclic nucleotide dependent protein kinases: in vivo characterization and structure/function relationships. Mass Spectrom Rev. 2008 Jul-Aug;27(4):331-53. doi: 10.1002/mas.20166.
- Smidt MP, Burbach JP (2007). How to make a mesodiencephalic dopaminergic neuron. Nat Rev Neurosci. 2007 Jan;8(1):21-32. Erratum in: Nat Rev Neurosci. 2007 Feb;8(2):160.
- Steegborn C (2014). Structure, mechanism, and regulation of soluble adenylyl cyclases - similarities and differences to transmembrane adenylyl cyclases. Biochim Biophys Acta. 2014 Dec;1842(12 Pt B):2535-47. doi: 10.1016/j.bbadis.2014.08.012. Epub 2014 Sep 2.
- Stephenson DT, Coskran TM, Kelly MP, Kleiman RJ, Morton D, O'Neill SM, Schmidt CJ, Weinberg RJ, Menniti FS (2012). The distribution of phosphodiesterase 2A in the rat brain. Neuroscience. 2012 Dec 13;226:145-55. doi: 10.1016/j.neuroscience.2012.09.011. Epub 2012 Sep 19.
- Stephenson DT, Coskran TM, Wilhelms MB, Adamowicz WO, O'Donnell MM, Muravnick KB, Menniti FS, Kleiman RJ, Morton D (2009). Immunohistochemical Localization of Phosphodiesterase 2A in Multiple Mammalian Species. J Histochem Cytochem. 2009 Oct;57(10):933-49. doi: 10.1369/jhc.2009.953471. Epub 2009 Jun 8.
- Tekin I, Roskoski R Jr, Carkaci-Salli N, Vrana KE (2014). Complex molecular regulation of tyrosine hydroxylase. J Neural Transm (Vienna). 2014 Dec;121(12):1451-81. doi: 10.1007/s00702-014-1238-7. Epub 2014 May 28.
- Upadhya MA, Shelkar GP, Subhedar NK, Kokare DM (2016). CART modulates the effects of levodopa in rat model of Parkinson's disease. Behav Brain Res. 2016 Mar 15;301:262-72. doi: 10.1016/j.bbr.2015.12.031. Epub 2016 Jan 8.
- Valentino MA, Lin JE, Snook AE, Li P, Kim GW, Marszalowicz G, Magee MS, Hyslop T, Schulz S, Waldman SA (2011). A uroguanylin-GUCY2C endocrine axis regulates feeding in mice. J Clin Invest. 2011 Sep;121(9):3578-88. doi: 10.1172/JCI57925. Epub 2011 Aug 25.
- Zaccolo M, Movsesian MA (2007). cAMP and cGMP signaling cross-talk: role of phosphodiesterases and implications for cardiac pathophysiology. Circ Res. 2007 Jun 8; 100(11): 1569-78.

**Table 2. Antibodies employed in western blotting (blue) and immunocytochemistry (green). The mouse and human GUCY2C were detected with the goat and mouse primary antibodies, respectively.**

| **Compound** | **Conc.** | **Function** |
|---|---|---|
| 8-Bromo-cGMP | 500µM | cGMP analogue and PKG activator |
| Guanylin | 10µM | GUCY2C ligand |
| Uroguanylin | 1µM | GUCY2C ligand |
| IBMX | 100µM | General phosphodiesterase inhibitor |
| Table 1. Example of a concentration, or concentration range and a function of different compounds used in the present invention | | |

| Antibody | Dilution | Species |
|---|---|---|
| GUCY2C | 1:170 | Mouse |
| GUCY2C | 1:170 | Goat |
| Total Th | 1:1,000 | Sheep |
| Total Th | 1:1,000 | Rabbit |
| P-Ser40 | 1:1,000 | Rabbit |
| Actin | 1:3,000 | Mouse |

| Primary Antibody | Dilution | Secondary Antibody |
|---|---|---|
| GUCY2C Goat | 1:100 | Donkey α Goat 488 |
| Total Th Rabbit | 1:1,000 | Goat α Rabbit 488 |

## Claims

1. A GUCY2C agonist for use in the treatment of an individual that has Parkinson's disease.

2. The GUCY2C agonist of claim 1, further comprising administering a PDE inhibitor to the individual.

3. The GUCY2C agonist of claim 1 or claim 2, wherein the individual has Parkinson's disease stage 1, 2, 3 or 4.

4. The GUCY2C agonist of any one of claims 1-3, wherein the agonist is guanylin or a functional derivative thereof.

5. The GUCY2C agonist of any one of claims 1-4, wherein the treatment further comprises a administering a PDE inhibitor to the individual.

6. A method of increasing dopamine production by a dopaminergic cell, the method comprising increasing signaling by Guanylate Cyclase 2C (GUCY2C) in said cell.

7. A method of increasing the level of phosphorylation of Ser40 of tyrosine hydroxylase in a dopaminergic cell, the method comprising increasing signaling by GUCY2C in said cell.

8. The method of claim 6 or claim 7, wherein the signaling by GUCY2C is increased by contacting said cell with a GUCY2C agonist.

9. The method of claim 8, wherein said agonist is guanylin or a functional derivative thereof.

10. The method of any one of claims 6-9, further comprising providing said cell with a PDE inhibitor.

11. The method according to any one of claims 6-10, wherein the cell is a dopaminergic neuron, preferably a midbrain dopaminergic neuron.

12. An isolated or recombinant human cell comprising ectopic expression of human GUCY2C and/or ectopic expression of human tyrosine hydroxylase.

13. The isolated or recombinant human cell of claim 12 comprising ectopic expression of human GUCY2C and ectopic expression of human tyrosine hydroxylase.

14. A method for identifying a candidate compound for modifying dopamine production by a dopaminergic cell, the method comprising culturing a cell according to claim 12 or claim 13; contacting said cell with the candidate compound and determining the activity of tyrosine hydroxylase in said cell.

15. The method of claim 14, wherein the level of phosphorylation of Ser40 of tyrosine hydroxylase is determined.

16. Use of a GUCY2C gene, an RNA or a protein encoded by the gene as a target for identifying a compound that is active in modulating tyrosine hydroxylase activity in a dopaminergic cell.

17. A method of treatment of an individual that has Parkinson's disease, or is at risk of developing the disease, the method comprising administering a GUCY2C agonist to the individual in need thereof.
